# EUROPÄISCHE PATENTSCHRIFT

(11) **EP 1 257 542 B1**
(45) Veröffentlichungstag und Bekanntmachung des Hinweises auf die Patenterteilung: **01.12.2004**
(21) Anmeldenummer: 01951142.7
(22) Anmeldetag: 29.01.2001
(51) Int. Cl.: C07D 249/08

(54) **VERFAHREN ZUR HERSTELLUNG VON 2-(1,2,4-TRIAZOL-1-YL)-ETHANOLEN**
METHOD FOR PRODUCING 2-(1,2,4-TRIAZOL-1-YL)-ETHANOLS
PROCEDE DE PRODUCTION DE 2-(1,2,4-TRIAZOL-1-YL)-ETHANOLS

(30) Priorität: 09.02.2000 DE 10005572
(43) Veröffentlichungstag der Anmeldung: 20.11.2002
(73) Patentinhaber: Bayer CropScience AG, 40789 Monheim (DE)
(72) Erfinder: HIMMLER, Thomas, 51519 Odenthal (DE)
(86) Internationale Anmeldenummer: PCT/EP2001/000908
(87) Internationale Veröffentlichungsnummer: WO 2001/058884

(56) Entgegenhaltungen:
- EP-A- 0 502 307
- DE-A- 4 030 039
- U. BECHSTEIN ET AL: "Efficient syntheses of omega-chloro-, omega-imido- and omega-aminoalkyl-1,2,4-triazoles from N-acyl-formamidinium salts or N-acylformamides and hydrazines" ARCHIV DER PHARMAZIE, Bd. 325, Nr. 8, 1992, Seiten 519-529, XP000997749 WEINHEIM

## Beschreibung

Die vorliegende Erfindung betrifft ein neues Verfahren zur Herstellung von 2-(1,2,4-Triazol-1-yl)-ethanolen, die mikrobizide, insbesondere fungizide Eigenschaften besitzen.

Es ist bereits bekannt geworden, dass zahlreiche 2-(1,2,4-Triazol-1-yl)-ethanole hergestellt werden können, indem man entsprechend substituierte Oxirane mit 1,2,4-Triazol in Gegenwart einer Base und eines Verdünnungsmittels umsetzt. Nachteilig an diesem Verfahren ist jedoch, dass außer den gewünschten 1,2,4-Triazol-1-yl-Verbindungen (= "asymmetrisches Triazol") auch unterschiedlich große Anteile an störenden 1,3,4-Triazol-1-yl-Derivaten (= "symmetrisches Triazol") erhalten werden (vgl. Tetrahedron Lett. **30** (1989) 4013-4016). Dies mindert naturgemäß die Ausbeute an 1,2,4-Triazol-1-yl-Derivat. Außerdem erschwert die Abtrennung der störenden 1,3,4-Triazol-1-yl-Verbindung die Aufarbeitung, so dass sich häufig die Ausbeute an 1,2,4-Triazol-1-yl-Derivat weiter verringert.

Es ist ferner bereits bekannt geworden, dass sich in 1-Position substituierte 1,2,4-Triazole durch Umsetzung von substituierten Hydrazinen mit [2-Aza-3-(dimethylamino)-2-propen-1-yliden]-dimethylimmoniumchlorid ("Gold's Reagenz") erhalten lassen (vgl. Angew. Chem. **72** (1960) 956-959). Die Ausbeuten sind jedoch nicht befriedigend. Nachteilig ist im übrigen der Zwangsanfall von 2 Mol Dimethylamin pro Mol Triazolyl-Derivat.

Weiterhin ist bereits bekannt geworden, dass in 1-Stellung substituierte 1,2,4-Triazole durch Umsetzung von Hydrazinderivaten mit Formamidinacetat erhalten werden können (vgl. "N-C-N Chemicals; Formamidine; Building block for heterocycles and intermediates", Firmenschrift der SKW Trostberg AG, 1989). So lässt sich 2-(1-Chlor-cyclopropyl)-1-(2-chlor-phenyl)-3-(1,2,4-triazol-1-yl)-propan-2-ol synthetisieren, indem man 2-(1-Chlor-cyclopropyl)-2-(2-chlorbenzyl)-oxiran mit Hydrazinhydrat umsetzt und das dabei entstehende 2-(1-Chlor-cycloprop-1-yl)-3-(2-chlorphenyl)-2-hydroxy-propyl-hydrazin dann mit Formamidinacetat zur Reaktion bringt (vgl. DE-A 40 30 039). Ungünstig ist aber, dass die Ausbeute an dem gewünschten Zielprodukt relativ niedrig ist.

Es wurde nun gefunden, dass sich 2-(1,2,4-Triazol-1-yl)-ethanole der Formel 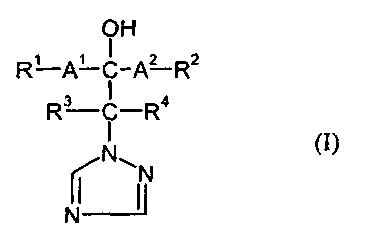 in welcher
- A¹ und A²: unabhängig voneinander für eine direkte Bindung, für gegebenenfalls durch Halogen substituiertes Alkandiyl, für gegebenenfalls durch Halogen substituiertes Alkendiyl, für gegebenenfalls durch Halogen substituiertes Alkindiyl oder für Alkandiyl, in dem eine Methylengruppe durch Sauerstoff ersetzt ist, stehen,
- R¹ und R²: unabhängig voneinander für Wasserstoff, für gegebenenfalls substituiertes Cycloalkyl oder für gegebenenfalls substituiertes Aryl stehen und
- R³ und R⁴: unabhängig voneinander für Wasserstoff oder für gegebenenfalls substituiertes Alkyl stehen oder
- R³ und R⁴: gemeinsam mit dem Kohlenstoffatom, an das sie gebunden sind, für gegebenenfalls substituiertes Cycloalkyl stehen,
oder
- R¹, A¹ und R³: gemeinsam mit den Kohlenstoffatomen, an die sie gebunden sind, für Cycloalkyl stehen,
- A²: für eine direkte Bindung, für gegebenenfalls durch Halogen substituiertes Alkandiyl, für gegebenenfalls durch Halogen substituiertes Alkendiyl, für gegebenenfalls durch Halogen substituiertes Alkindiyl oder für Alkandiyl, in dem eine Methylengruppe durch Sauerstoff ersetzt ist, steht,
- R²: für Wasserstoff, für gegebenenfalls substituiertes Cycloalkyl oder für gegebenenfalls substituiertes Aryl steht und
- R⁴: für Wasserstoff oder für gegebenenfalls substituiertes Alkyl steht,
oder
R³ und R⁴ für Wasserstoff stehen und die Gruppen
R¹-A¹- und R²-A²- gemeinsam mit dem Kohlenstoffatom, an das sie gebunden sind, für einen Rest der Formel 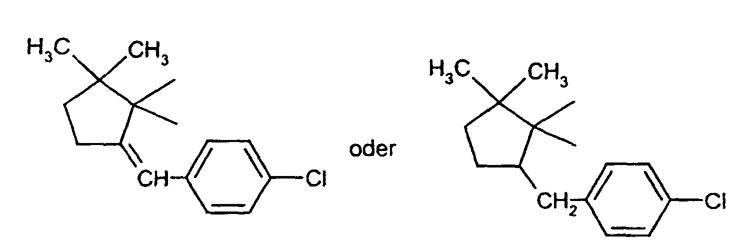 stehen, herstellen lassen, indem man Hydrazin-Derivate der Formel 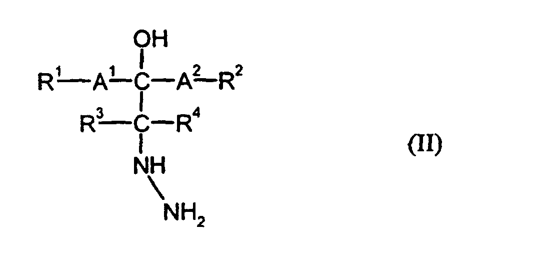 in welcher
A¹, A², R¹, R², R³ und R⁴ die oben angegebenen Bedeutungen haben,
mit N-Dihalogenmethyl-formamidiniumhalogenid der Formel 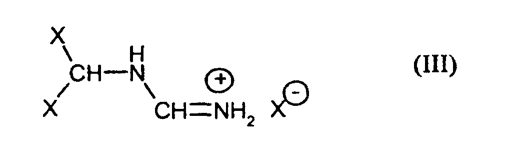 in welcher
- X: für Chlor oder Brom steht,
gegebenenfalls in Gegenwart eines Verdünnungsmittels umsetzt.

Es ist als ausgesprochen überraschend zu bezeichnen, dass sich 2-(1,2,4-Triazol-1-yl)-ethanole der Formel (I) nach dem erfindungsgemäßen Verfahren in glatter Reaktion herstellen lassen, denn aufgrund des vorbekannten Standes der Technik war mit störenden Nebenreaktionen und mit Zersetzung von N-Dihalogenmethyl-formamidinium-halogenid der Formel (III) zu rechnen. So geht aus Ber. dtsch. Chem. Ges. 16 (1883) 308-311 hervor, dass N-Dichlormethyl-formarnidinium-chiorid durch Wasser oder Alkohole zersetzt wird. Weiterhin wurde in Chem. Ber. 35, (1902) 2496-2511 beschrieben, dass ein Äquivalent N-Dichlormethylformamidiniumchlorid mit zwei Äquivalcnten eines primären Amins der Formel R-NH₂ leicht und vollständig unter Abspaltung von drei Äquivalenten Chlorwasserstoff zu einem Äquivalent Formamidin und einem Äquivalent an disubstituiertem Formamidin der Formel R-N=CH-NH-R reagiert. Bei der Umsetzung von o-Phenylendiamin mit N-Dichlormethyl-formamidinium-chlorid wird Benzimidazol erhalten (vgl. Chem. Ber. 35 (1902), 2496-2511). Phenylhydrazin reagiert mit N-Dichlorrnethylformamidinium-chlorid wie primäres Amin, allerdings wird das entstandene Formamidin-Derivat noch durch Luftsauerstoff oder ein drittes Äquivalent Phenylhydrazin zu Diphenylformazan der Formel Ph-N=N-CH=N-NH-Ph oxidiert (vgl. Chem. Ber. 35 (1902), 2496-2511). Die Bildung von 1-Phenyl-1,2,4-triazol aus Phenylhydrazin und N-Dichlorrnethyl-formamidinium-chlorid wird nicht berichtet. Unter Berücksichtigung dieser bekannten Reaktionen war zweifellos nicht zu erwarten, dass die erfindungsgemäße Umsetzung die gewünschten Zielprodukte in hoher Ausbeute liefern würde.

Das erfindungsgemäße Verfahren zeichnet sich durch eine Reihe von Vorteilen aus. So ermöglicht es, wie schon erwähnt, die Synthese von 2-(1,2,4-Triazol-1-yl)-ethanolen der Formel (I) in hoher Ausbeute, und zwar frei von den entsprechenden "symmetrischen" Triazol-Derivaten. Günstig ist auch, dass die benötigten Ausgangsstoffe und Reaktionskomponenten einfach herstellbar und auch in größeren Mengen verfügbar sind. Ein weiterer Vorteil besteht schließlich darin, dass die Ausbeuten höher liegen als bei vergleichbaren Reaktionen von Hydrazinen mit Formamidinacetat oder "Gold's Reagenz". Günstig gegenüber der Verwendung von "Gold's Reagenz" ist außerdem, dass im Zuge der erfindungsgemäßen Umsetzungen mit N-Dihalogenmethyl-formamidinium-halogenid nur Halogenwasserstoff und Ammoniak anfallen.

Verwendet man [1-(2-Chlorphenyl)-2-(1-chlor-cyclopropyl)-2-hydroxy]-propylhydrazin-hydrochlorid und N-Dichlormethyl-forrnarnidinium-chlorid als Ausgangssubstanzen, so kann der Verlauf des erfindungsgemäßen Verfahrens durch das folgende Formelschema veranschaulicht werden.

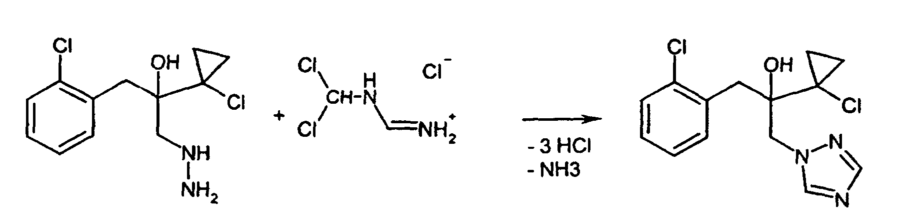

Die bei der Durchführung des erfindungsgemäßen Verfahrens als Ausgangsstoffe benötigten Hydrazin-Derivate sind durch die Formel (II) allgemein definiert. Bevorzugt einsetzbar sind Hydrazin-Derivate der Formel (II), in denen
- A¹: für eine direkte Bindung, für gegebenenfalls durch Halogen substituiertes, geradkettiges oder verzweigtes Alkandiyl mit 1 bis 4 Kohlenstoffatomen, für gegebenenfalls durch Halogen substituiertes, geradkettiges oder verzweigtes Alkendiyl mit 2 bis 4 Kohlenstoffatomen, für gegebenenfalls durch Halogen substituiertes, geradkettiges oder verzweigtes Alkindiyl mit 2 bis 4 Kohlenstoffatomen oder für geradkettiges oder verzweigtes Alkandiyl, in dem eine Methylengruppe durch Sauerstoff ersetzt ist, mit 2 bis 4 Kettengliedern steht,
- A²: für eine direkte Bindung, für gegebenenfalls durch Halogen substituiertes, geradkettiges oder verzweigtes Alkandiyl mit 1 bis 6 Kohlenstoffatomen, für gegebenenfalls durch Halogen substituiertes, geradkettiges oder verzweigtes Alkendiyl mit 2 bis 6 Kohlenstoffatomen oder für gegebenenfalls durch Halogen substituiertes, geradkettiges oder verzweigtes Alkindiyl mit 2 bis 6 Kohlenstoffatomen steht,
- R¹: für gegebenenfalls einfach bis dreifach, gleichartig oder verschieden durch Fluor, Chlor, Brom und/oder Alkyl mit 1 bis 4 Kohlenstoffatomen substituiertes Cycloalkyl mit 3 bis 7 Kohienstoffatomen steht oder für Aryl mit 6 bis 10 Kohlenstoffatomen steht, das einfach bis dreifach, gleichartig oder verschieden substituiert sein kann durch Fluor, Chlor, Brom, Alkyl mit 1 bis 4 Kohlenstoffatomen, Cyano, Nitro, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 9 Fluor-, Chlor- und/oder Bromatomen, Halogenalkoxy mit 1 bis 4 Kohlenstoffatomen und 1 bis 9 Fluor-, Chlor- und/oder Bromatomen oder Halogenalkylthio mit 1 bis 4 Kohlenstoffatomen und 1 bis 9 Fluor-, Chlor- und/oder Bromatomen,
- R²: für Wasserstoff oder für gegebenenfalls einfach bis dreifach, gleichartig oder verschieden durch Fluor, Chlor, Brom und/oder Alkyl mit 1 bis 4 Kohlenstoffatomen substituiertes Cycloalkyl mit 3 bis 7 Kohlenstoffatomen steht oder
für Aryl mit 6 bis 10 Kohlenstoffatomen steht, das einfach bis dreifach, gleichartig oder verschieden substituiert sein kann durch Fluor, Chlor, Brom, Alkyl mit 1 bis 4 Kohlenstoffatomen, Cyano, Nitro, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 9 Fluor-, Chlor- und/oder Bromatomen, Halogenalkoxy mit 1 bis 4 Kohlenstoffatomen und 1 bis 9 Fluor-, Chlor- und/oder Bromatomen oder Halogenalkylthio mit 1 bis 4 Kohlenstoffatomen und 1 bis 9 Fluor-, Chlor- und/oder Bromatomen, und
- R³ und R⁴: unabhängig voneinander für Wasserstoff oder gegebenenfalls einfach bis dreifach, gleichartig oder verschieden durch Fluor, Chlor, Brom oder Alkoxy mit 1 oder 2 Kohlenstoffatomen substituiertes Alkyl mit 1 bis 4 Kohlenstoffatomen stehen oder
- R³ und R⁴: gemeinsam mit dem Kohlenstoffatom, an das sie gebunden sind, für gegebenenfalls einfach bis dreifach, gleichartig oder verschieden durch Fluor, Chlor, Brom und/oder Alkyl mit 1 bis 4 Kohlenstoffatomen substituiertes Cycloalkyl mit 3 bis 6 Kohlenstoffatomen stehen,
oder
- R¹, A¹ und R³: gemeinsam mit den Kohlenstoffatomen, an die sie gebunden sind, für Cycloalkyl mit 3 bis 6 Kohlenstoffatomen stehen,
- A²: für eine direkte Bindung steht,
- R²: für Wasserstoff oder für gegebenenfalls einfach bis dreifach, gleichartig oder verschieden durch Fluor, Chlor, Brom und/oder Alkyl mit 1 bis 4 Kohlenstoffatomen substituiertes Cycloalkyl mit 3 bis 7 Kohlenstoffatomen steht oder
für Aryl mit 6 bis 10 Kohlenstoffatomen steht, das einfach bis dreifach, gleichartig oder verschieden substituiert sein kann durch Fluor, Chlor, Brom, Alkyl mit 1 bis 4 Kohlenstoffatomen, Cyano, Nitro, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 9 Fluor-, Chlor- und/oder Bromatomen, Halogenalkoxy mit 1 bis 4 Kohlenstoffatomen und 1 bis 9 Fluor-, Chlor- und/oder Bromatomen oder Halogenalkylthio mit 1 bis 4 Kohlenstoffatomen und 1 bis 9 Fluor-, Chlor- und/oder Bromatomen, und
- R⁴: für Wasserstoff oder gegebenenfalls einfach bis dreifach, gleichartig oder verschieden durch Fluor, Chlor, Brom oder Alkoxy mit 1 oder 2 Kohlenstoffatomen substituiertes Alkyl mit 1 bis 4 Kohlenstoffatomen steht,
oder
- R³ und R⁴: für Wasserstoff stehen und die Gruppen
- R¹-A¹- und R²-A²-: gemeinsam mit dem Kohlenstoffatom, an das sie gebunden sind, für einen Rest der Formel 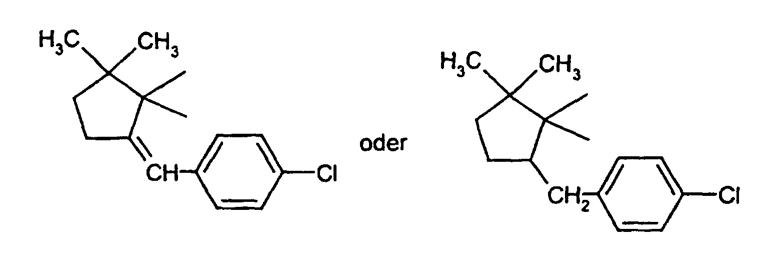
stehen.

Besonders bevorzugt sind Hydrazin-Derivate der Formel (II), in denen
- A¹: für eine Einfachbindung, für Methylen, Ethan-1,1-diyl, Ethan-1,2-diyl, Ethen-1,2-diyl, Ethin-1,2-diyl oder -O-CH₂ steht, wobei die CH₂-Gruppe mit dem Carbinol-Kohlenstoffatom verbunden ist,
- A²: für eine direkte Bindung steht oder für jeweils gegebenenfalls einfach oder zweifach durch Fluor und/oder Chlor substituiertes Methylen, Ethan-1,1-diyl, Ethan-1,2-diyl, Propan-1,1-diyl, Propan-1,2-diyl, Propan-1,3-diyl, Propan-2,2-diyl, Butan-1,1-diyl, Butan-1,2-diyl, Butan-1,3-diyl, Butan-1,4-diyl, Butan-2,2-diyl, 2-Methyl-propan-1,2-diyl, Ethylen-1,2-diyl oder Ethin-1,2-diyl steht,
- R¹: für gegebenenfalls einfach bis dreifach, gleichartig oder verschieden durch Fluor, Chlor, Brom, Methyl, Ethyl, n-Propyl, i-Propyl, n-Butyl, iso-Butyl, sek.-Butyl oder tert.-Butyl substituiertes Cycloalkyl mit 3 bis 6 Kohlenstoffatomen steht oder
für Phenyl oder Naphthyl steht, das jeweils einfach bis dreifach, gleichartig oder verschieden substituiert sein kann durch Fluor, Chlor, Brom, Cyano, Nitro, Methyl, Ethyl, n-Propyl, i-Propyl, n-Butyl, sek.-Butyl, iso-Butyl, tert.-Butyl, Methoxy, Ethoxy, Methylthio, Ethylthio, Trifluormethyl, Trifluormethoxy, Difluormethoxy, Trifluormethylthio und/oder Difluormethylthio,
- R²: für Wasserstoff oder für gegebenenfalls einfach bis dreifach, gleichartig oder verschieden durch Fluor, Chlor, Brom, Methyl, Ethyl, n-Propyl, i-Propyl, n-Butyl, iso-Butyl, sek.-Butyl oder tert.-Butyl substituiertes Cycloalkyl mit 3 bis 6 Kohlenstoffatomen steht oder
für Phenyl oder Naphthyl steht, das jeweils einfach bis dreifach, gleichartig oder verschieden substituiert sein kann durch Fluor, Chlor, Brom, Cyano, Nitro, Methyl, Ethyl, n-Propyl, i-Propyl, n-Butyl, sek.-Butyl, iso-Butyl, tert.-Butyl, Methoxy, Ethoxy, Methylthio, Ethylthio, Trifluormethyl, Trifluormethoxy, Difluormethoxy, Trifluormethylthio und/oder Difluormethylthio,
- R³ und R⁴: unabhängig voneinander für Wasserstoff, Methyl oder Ethyl stehen oder
- R³ und R⁴: gemeinsam mit dem Kohlenstoffatom, an das sie gebunden sind, für Cyclopropyl, Cyclobutyl, Cyclopentyl oder Cyclohexyl stehen
oder
- R¹, A¹ und R³: gemeinsam mit den Kohlenstoffatomen, an die sie gebunden sind, für Cyclopropyl, Cyclobutyl, Cyclopentyl oder Cyclohexyl stehen,
- A²: für eine direkte Bindung steht,
- R²: für Wasserstoff oder
für gegebenenfalls einfach bis dreifach, gleichartig oder verschieden durch Fluor, Chlor, Brom, Methyl, Ethyl, n-Propyl, i-Propyl, n-Butyl, iso-Butyl, sek.-Butyl oder tert.-Butyl substituiertes Cycloalkyl mit 3 bis 6 Kohlenstoffatomen steht, oder
für Phenyl oder Naphthyl steht, das jeweils einfach bis dreifach, gleichartig oder verschieden substituiert sein kann durch Fluor, Chlor, Brom, Cyano, Nitro, Methyl, Ethyl, n-Propyl, i-Propyl, n-Butyl, sek.-Butyl, iso-Butyl, tert.-Butyl, Methoxy, Ethoxy, Methylthio, Ethylthio, Trifluormethyl, Trifluormethoxy, Difluonnethoxy, Trifluormethylthio und/oder Difluormethylthio und
- R⁴: für Wasserstoff, Methyl oder Ethyl steht,
oder
- R³ und R⁴: für Wasserstoff stehen und die Gruppen
- R¹-A¹- und R²-A²-: gemeinsam mit dem Kohlenstoffatom, an das sie gebunden sind, für einen Rest der Formel 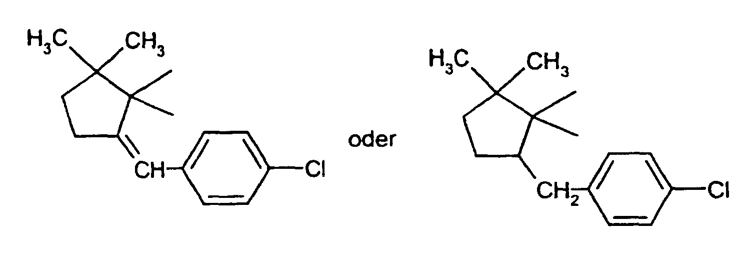
stehen.

Die Hydrazin-Derivate der Formel (II) sind bekannt oder lassen sich nach bekannten Methoden herstellen. So erhält man Hydrazin-Derivate der Formel (II), wenn man Oxirane der Formel 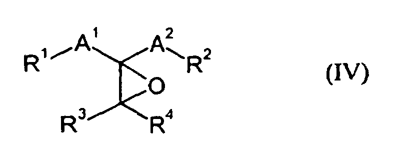 in welcher
A¹,A²,R¹,R²,R³ und R⁴ die oben angebenen Bedeutungen haben,
mit Hydrazin oder Hydrazinhydrat umsetzt (vgl. J. Org. Chem. 61 (1996) 4125; J. Amer. Chem. Soc. 99 (1977) 1172; Bull. Soc. Chim. Fr. 1947 850; und Bull. Soc. Chim. Fr. 1939, 708).

Die Oxirane der Formel (IV) sind bekannt oder lassen sich nach bekannten Methoden herstellen.

Die Hydrazinderivate der Formel (II) können entweder als freie Basen oder in Form ihrer Salze wie beispielsweise ihrer Hydrochloride, Hydrobromide oder Hydrogensulfate eingesetzt werden. Bevorzugt werden die Hydrochloride eingesetzt.

Die weiterhin zur Durchführung des erfindungsgemäßen Verfahrens als Ausgangsstoffe benötigten N-Dihalogenmethyl-formamidiniumhalogenide sind durch die Formel (III) allgemein definiert. In dieser Formel steht X vorzugsweise für Chlor.

Die N-Dihalogenmethyl-formamidiniumhalogenide (III) sind bekannt oder lassen sich nach bekannten Methoden herstellen. So erhält man N-Dihalogenmethyl-formamidinium-halogenide der Formel (III), indem man 2 Äquivalente Blausäure mit 3 Äquivalenten Halogenwasserstoff umsetzt. Die N-Dihalogenmethyl-formamidinium-halogenide werden auch als Sesquihydrohalogenide der Blausäure bezeichnet. N-Dichlormethyl-formamidiniumchlorid kann beispielsweise durch Einleiten von trockenem Chlorwasserstoff in eine auf ca. -10°C gekühlte Lösung von Blausäure in einem Verdünnungsmittel, wie beispielsweise Essigsäureethylester, hergestellt werden (vgl. Ber. dtsch. Chem. Ges. **16** (1883) 308-311). N-Dichlormethyl-formamidiniumchlorid kann durch die Formel 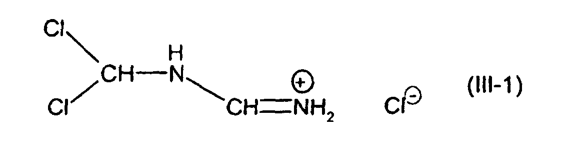 dargestellt werden. Andere Strukturen sind jedoch auch möglich (vgl. Chem. Ber. 99 (1966), 431-444). Der Einfachheit halber wird im folgenden nur die Formel (III-1) verwendet, ohne dass damit eine Entscheidung über die tatsächliche Struktur des Sesquihydrochlorids der Blausäure getroffen wird.

Als Verdünnungsmittel kommen bei der Durchführung des erfindungsgemäßen Verfahrens prinzipiell alle üblichen inerten, organischen Solventien in Frage. Vorzugsweise verwendbar sind Carbonsäureester, wie Ameisensäuremethylester, Ameisensäureethylester, Essigsäureethylester und Essigsäuremethylester, ferner Ether, wie Tertiärbutyl-methylether und 1,4-Dioxan, weiterhin N,N-disubstituierte Säureamide, wie N,N-Dimethylformamid, N,N-Dimethylacetamid und N-Methylpyrrolidon, und außerdem aromatische oder aliphatische Kohlenwasserstoffe, wie Toluol, Xylol, Cyclohexan und Methylcyclohexan. Besonders bevorzugt sind Essigsäuremethylester, Essigsäureethylester und Toluol.

Die Reaktionstemperaturen können bei der Durchführung des erfindungsgemäßen Verfahrens innerhalb eines größeren Bereiches variiert werden. Häufig ist die Obergrenze des Temperaturbereichs durch die Zersetzungstemperatur des Hydrazinderivats der Formel (II) gegeben. Im allgemeinen arbeitet man bei Temperaturen zwischen 20 und 200°C, vorzugsweise zwischen 30 und 150°C.

Bei der Durchführung des erfindungsgemäßen Verfahrens arbeitet man im allgemeinen bei Atmosphärendruck. Es ist aber auch möglich, unter erhöhtem oder vermindertem Druck zu arbeiten.

Bei der Durchführung des erfindungsgemäßen Verfahrens kann das Verhältnis, in dem die Reaktionskomponenten eingesetzt werden, innerhalb eines größeren Bereiches variiert werden. Aus ökonomischen Gründen setzt man auf 1 Mol an Hydrazin-Derivat der Formel (II) oder an dessen Salz mindestens 1 Mol an N-Dihalogenmethyl-formamidinium-halogenid der Formel (III) ein. Es ist aber auch möglich, weniger als 1 Mol an N-Dihalogenmethyl-formamidinium-halogenid zu verwenden. Vorzugsweise setzt man N-Dihalogenmethylformamidinium-halogenid der Formel (III) in einem Überschuß von 1 bis 30 Mol-%, besonders bevorzugt von 5 bis 20 Mol-%, ein. Die Aufarbeitung erfolgt nach üblichen Methoden. Im allgemeinen verfährt man in der Weise, dass man das Reaktionsgemisch mit Wasser und einem mit Wasser wenig mischbaren organischen Lösungsmittel versetzt, die organische Phase abtrennt, die wässrige Phase mit einem organischen Lösungsmittel extrahiert, die vereinigten organischen Phasen trocknet und einengt. Gegebenenfalls noch enthaltene Verunreinigungen können nach üblichen Methoden, zum Beispiel durch Umkristallisation, Destillation oder auf chromatographischem Wege, entfernt werden.

Die nach dem erfindungsgemäßen Verfahren herstellbaren 2-(1,2,4-Triazol-1-yl)-ethanole der Formel (I) sind bekannt (vgl. EP-A 0 040 345 und EP-A 0 297 345).

Die 2-(1,2,4-Triazol-1-yl)-ethanole der Formel (I) besitzen mikrobizide, insbesondere fungizide Eigenschaften.

Die Durchführung des erfindungsgemäßen Verfahrens wird durch die folgenden Beispiele veranschaulicht.

### Herstellungsbeispiele:

### Beispiel 1

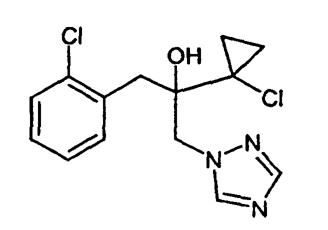

In 25 ml Essigester werden 3,12 g 2-(1-Chlor-cycloprop-1-yl)-3-(2-chlorphenyl)-2-hydroxy-propyl-1-hydrazin-Hydrochlorid, dessen Gehalt zu 95% bestimmt wurde (= 9,5 mmol), bei Raumtemperatur suspendiert. Man gibt 2 g (12 mmol) N-Dichlormethyl-formamidiniumchlorid hinzu und rührt 5 Stunden bei 45°C. Das Reaktionsgemisch wird dann bei Raumtemperatur mit 20 ml Wasser und 30 ml Essigsäureethylester versetzt. Die wässrige Phase wird abgetrennt und mit 15 ml Essigsäureethylester ausgeschüttelt. Die vereinigten organischen Phasen werden über Natriumsulfat getrocknet und unter vermindertem Druck eingeengt. Man erhält auf diese Weise 3,59 g eines Produktes, das zu 82,3% (HPLC) aus 2-(1-Chlor-cyclopropyl)-1-(2-chlorphenyl)-3-(1,2,4-triazol-1-yl)-propan-2-ol besteht. Die Ausbeute errechnet sich danach zu 99,6 % der Theorie.

### Beispiel 2

Es wird vorgegangen wie in Beispiel 1, jedoch werden nur 1,72 g (10,5 mmol) N-Dichlormethyl-forrnamidiniumchlorid eingesetzt. Es werden 3,43 g eines Produktes erhalten, das zu 84,9% (HPLC) aus 2-(1-Chlor-cyclopropyl)-1-(2-chlorphenyl)-3-(1,2,4-triazol-1-yl)-propan-2-ol besteht. Die Ausbeute errechnet sich danach zu 98,2 % der Theorie.

### Beispiel 3

In 25 ml Toluol werden 10 mmol 2-(1-Chlor-cycloprop-1-yl)-3-(2-chlorphenyl)-2-hydroxy-propyl-1-hydrazin-Hydrochlorid bei Raumtemperatur suspendiert. Man gibt 1,72 g (10,5 mmol) N-Dichlormethyl-formamidiniumchlorid hinzu und rührt 5 Stunden unter Rückfluß. Das Reaktionsgemisch wird dann bei Raumtemperatur mit 20 ml Wasser und 30 ml Toluol versetzt. Die wässrige Phase wird abgetrennt und mit 15 ml Toluol ausgeschüttelt. Die vereinigten organischen Phasen werden über Natriumsulfat getrocknet und unter vermindertem Druck eingeengt. Man erhält auf diese Weise 3,47 g eines Produktes, das zu 89,9 % (HPLC) aus 2-(1-Chlor-cyclopropyl)-1-(2-chlorphenyl)-3-(1,2,4-triazol-1-yl)-propan-2-ol besteht. Die Ausbeute errechnet sich danach zu 100 % der Theorie.

### Beispiel 4

Es wird vorgegangen wie in Beispiel 2, jedoch wird Ameisensäureethylester anstelle von Essigsäureethylester als Lösungsmittel verwendet.

Die Ausbeute an 2-(1-Chlor-cyclopropyl)-1-(2-chlorphenyl)-3-(1,2,4-triazol-1-yl)-propan-2-ol beträgt 78 % der Theorie.

### Beispiel 5

Es wird vorgegangen wie in Beispiel 2, jedoch wird N,N-Dimethylformamid anstelle von Essigsäureethylester als Lösungsmittel verwendet.

Die Ausbeute an 2-(1-Chlor-cyclopropyl)-1-(2-chlorphenyl)-3-(1,2,4-triazol-1-yl)-propan-2-ol beträgt 63 % der Theorie.

### Beispiel 6

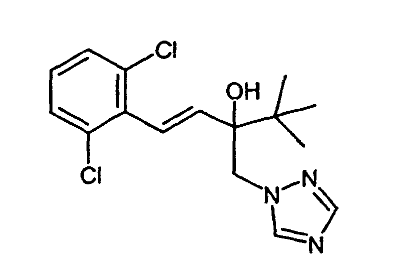

In 25 ml Essigsäureethylester werden 10 mmol 2-Tertiärbutyl-4-(2,6-dichlorphenyl)-2-hydroxy-but-3-en-1-hydrazin-Hydrochlorid suspendiert. Man gibt 2 g (12 mmol) N-Dichlormethyl-formamidiniumchlorid hinzu und rührt 5 Stunden bei 45°C. Das Reaktionsgemisch wird bei Raumtemperatur mit 20 ml Wasser und 30 ml Essigsäureethylester verrührt. Nach Phasentrennung wird die wässrige Phase nochmals mit 15 ml Essigsäureethylester ausgeschüttelt. Die vereinigten organischen Phasen werden über Natriumsulfat getrocknet und unter vermindertem Druck eingeengt. Es werden 3,06 g 3-Tertiärbutyl-1-(2,6-dichiorphenyl)-4-(1,2,4-triazol-1-yl)-but-1-en-3-ol erhalten (90 % der Theorie).

¹H-NMR(400 MHz, CDCl₃): δ = 1,15 (s; 9H), 4,55 - 4,64 (m; 2H), 6,16 (d, J = 16 Hz; 1H), 6,38 (d, J = 16 Hz; 1H), 6,98 (m; 1H), 7,15 (m; 2H), 8,25 (s; 1H), 9,04 (s; 1H) ppm.

### Beispiel 7

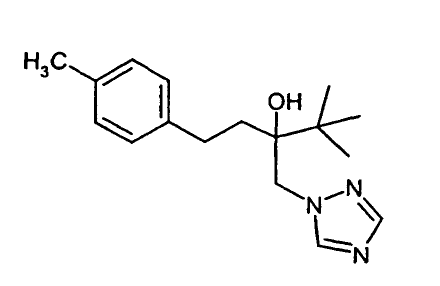

In 25 ml Essigsäureethylester werden 10 mmol 2-Tertiärbutyl-4-(4-methyl-phenyl)-2-hydroxy-butyl-1-hydrazin-Hydrochlorid suspendiert. Man gibt 2 g (12 mmol) N-Dichlormethyl-formamidiniumchlorid hinzu und rührt 5 Stunden bei 45°C. Das Reaktionsgemisch wird bei Raumtemperatur mit 20 ml Wasser und 30 ml Essigsäureethylester verrührt. Nach Phasentrennung wird die wässrige Phase nochmals mit 15 ml Essigsäureethylester ausgeschüttelt. Die vereinigten organischen Phasen werden über Natriumsulfat getrocknet und unter vermindertem Druck eingeengt. Es werden 2,06 g eines Öles erhalten, das nach HPLC zu 69 % aus 3-Tertiärbutyl-1-(4-methyl-phenyl)-4-(1,2,4-triazol-1-yl)-butan-3-ol besteht. Die Ausbeute errechnet sich danach zu 49 % der Theorie.
LC/MS (ESI positiv): MH⁺ = 288

### Beispiel 8

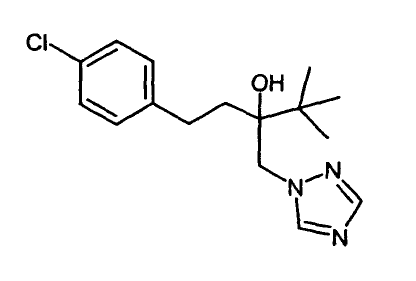

In 25 ml Essigsäureethylester werden 10 mmol 2-Tertiärbutyl-4-(4-chlor-phenyl)-2-hydroxy-butyl-1-hydrazin-Hydrochlorid suspendiert. Man gibt 2 g (12 mmol) N-Dichlormethyl-formamidiniumchlorid hinzu und rührt 5 Stunden bei 45°C. Das Reaktionsgemisch wird bei Raumtemperatur mit 20 ml Wasser und 30 ml Essigsäureethylester verrührt. Nach Phasentrennung wird die wässrige Phase nochmals mit 15 ml Essigsäureethylester ausgeschüttelt. Die vereinigten organischen Phasen werden über Natriumsulfat getrocknet und unter vermindertem Druck eingeengt. Es werden 3,1 g eines Feststoffes erhalten, der nach HPLC zu 96,7% aus 3-Tertiärbutyl-1-(4-chlor-phenyl)-4-(1,2,4-triazol-1-yl)-butan-3-ol besteht. Die Ausbeute errechnet sich danach zu 97% der Theorie.
¹H-NMR(400 MHz, d-DMSO): δ = 0,93 (s; 9H), 1,60 - 1,64 (m; 1H), 1,73 - 1,78 (m; 1H), 1,95 - 1,99 (m; 1H), 2,53 - 2,56 (m; 1H), 4,30 - 4,43 (m; 2H), 7,14 (m, 2H), 7,29 (m; 2H), 8,20 (s; 1H), 8,76 (s; 1H) ppm.

### Beispiel 9

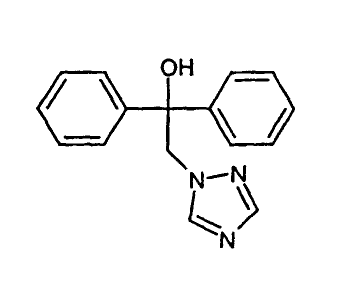

In 25 ml Essigsäureethylester werden 10 mmol 2,2-Diphenyl-2-hydroxy-ethyl-1-hydrazin-Hydrochlorid suspendiert. Man gibt 2 g (12 mmol) N-Dichlormethyl-formamidiniumchlorid hinzu und rührt 5 Stunden bei 45°C. Das Reaktionsgemisch wird bei Raumtemperatur mit 20 ml Wasser und 15 ml Essigsäureethylester verrührt. Der Feststoff wird abfiltriert, mit Wasser und Essigsäureethylester gewaschen und getrocknet. Es werden 2,0 g eines Feststoffes erhalten, der nach HPLC zu > 99% aus 1,1-Diphenyl-2-(1,2,4-triazol-1-yl)-ethan-1-ol besteht. Die Ausbeute errechnet sich danach zu 66 % der Theorie.

Fp. : 202-203 °C.

Aus den vereinigten organischen Phasen werden nach dem Trocknen über Natriumsulfat und Einengen im Vakuum weitere 0,92 g Produkt erhalten, das nach HPLC 67 %ig ist (= 0,62 g = 23 % der Theorie).

¹H-NMR(400 MHz, CDCl₃): δ = 4,90 (s; 2H), 7,25 - 7,28 (m; 2H), 7,30 - 7,33 (m; 4H), 7,40 - 7,42 (m; 4H), 7,69 (s; 1H), 7,83 (s; 1H) ppm.

### Beispiel 10

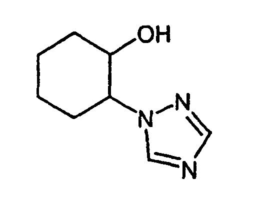

In 25 ml Essigsäureethylester werden 10 mmol 2-Hydroxy-cyclohex-1-yl-hydrazin gelöst. Man gibt 2 g (12 mmol) N-Dichlormethyl-formamidiniumchlorid hinzu und rührt 5 Stunden bei 45°C. Das Reaktionsgemisch wird bei Raumtemperatur mit 20 ml Wasser und 30 ml Essigsäureethylester verrührt. Man trennt die wässrige Phase ab, stellt sie mit gesättigter wässriger Natriumbicarbonatlösung neutral und schüttelt dreimal mit je 30 ml Essigsäureethylester aus. Die organische Phase wird über Natriumsulfat getrocknet und im Vakuum eingeengt. Man erhält 0,48 g gelblichen Feststoff, der nach HPLC zu 92 % aus 2-(1,2,4-Triazol-1-yl)-cyclohexanol besteht. Die Ausbeute errechnet sich danach zu 26 % der Theorie.

### Vergleichsbeispiel

### Herstellung bekannt aus DE-A 40 30 039

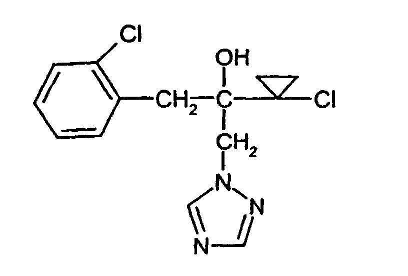

In ein Gemisch aus 50 g (1 Mol) Hydrazinhydrat und 200 ml n-Butanol werden bei Temperaturen zwischen 65 und 70°C unter Rühren 121,5 g (0,5 Mol) 2-(1-Chlorcyclopropyl)-2-(2-chlor-benzyl)-oxiran innerhalb von 2 Stunden eingetropft. Nach beendeter Zugabe wird noch weitere 2 Stunden bei 65 bis 70°C nachgerührt und dann auf 20°C abgekühlt. Man trennt die wässrige Phase ab, verrührt die verbleibende organische Phase mit 50 ml Wasser, trennt die organische Phase ab und engt diese organische Phase bei 70°C unter vermindertem Druck ein.

Der verbleibende Rückstand wird mit 200 ml Ethanol versetzt. Zu der entstehenden Lösung gibt man bei 70 bis 75°C unter Rühren portionsweise 114 g (1,1 Mol) Formamidinacetat hinzu. Nach beendeter Zugabe wird das Reaktionsgemisch noch 10 Stunden unter Rühren auf Temperaturen zwischen 75 und 80°C erhitzt. Anschließend arbeitet man auf, indem man das Reaktionsgemisch bei 70°C unter vermindertem Druck einengt. Man nimmt den verbleibenden Rückstand bei 70°C in 350 ml Methylcyclohexan auf und stellt nach Zugabe von 200 ml mit 45 gew.-%iger wässriger Natronlauge auf einen pH-Wert zwischen 7,5 und 8,0 ein. Die wässrige Phase wird abgetrennt, und die organische Phase wird zweimal mit je 200 ml Wasser bei 70°C gewaschen. Danach wird die organische Phase abgetrennt und langsam auf 20°C abgekühlt. Der dabei ausfallende Feststoff wird abgesaugt, zweimal mit je 100 ml Methylcyclohexan gewaschen und getrocknet. Man erhält auf diese Weise 117,5 g eines Produktes, das zu 85 % aus 2-(1-Chlor-cyclopropyl)-1-(2-chlorphenyl)-3-(1,2,4-triazol-1-yl)-propan-2-ol besteht.

Die Ausbeute errechnet sich danach zu 64 % der Theorie.

## Patentansprüche

1. Verfahren zur Herstellung von 2-(1,2,4-Triazol-1-yl)-ethanolen der Formel 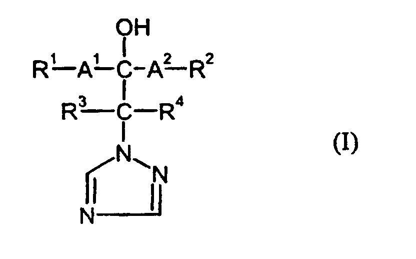 in welcher
A¹ und A² unabhängig voneinander für eine direkte Bindung, für gegebenenfalls durch Halogen substituiertes Alkandiyl, für gegebenenfalls durch Halogen substituiertes Alkendiyl, für gegebenenfalls durch Halogen substituiertes Alkindiyl oder für Alkandiyl, in dem eine Methylengruppe durch Sauerstoff ersetzt ist, stehen,
R¹ und R² unabhängig voneinander für Wasserstoff, für gegebenenfalls substituiertes Cycloalkyl oder für gegebenenfalls substituiertes Aryl stehen und
R³ und R⁴ unabhängig voneinander für Wasserstoff oder für gegebenenfalls substituiertes Alkyl stehen oder
R³ und R⁴ gemeinsam mit dem Kohlenstoffatom, an das sie gebunden sind, für gegebenenfalls substituiertes Cycloalkyl stehen,
oder
R¹, A¹ und R³ gemeinsam mit den Kohlenstoffatomen, an die sie gebunden sind, für Cycloalkyl stehen,
A² für eine direkte Bindung, für gegebenenfalls durch Halogen substituiertes Alkandiyl, für gegebenenfalls durch Halogen substituiertes Alkendiyl, für gegebenenfalls durch Halogen substituiertes Alkindiyl oder für Alkandiyl, in dem eine Methylengruppe durch Sauerstoff ersetzt ist, steht,
R² für Wasserstoff, für gegebenenfalls substituiertes Cycloalkyl oder für gegebenenfalls substituiertes Aryl steht und
R⁴ für Wasserstoff oder für gegebenenfalls substituiertes Alkyl steht,
oder
R³ und R⁴ für Wasserstoff stehen und die Gruppen
R¹-A¹- und R²-A²- gemeinsam mit dem Kohlenstoffatom, an das sie gebunden sind, für einen Rest der Formel 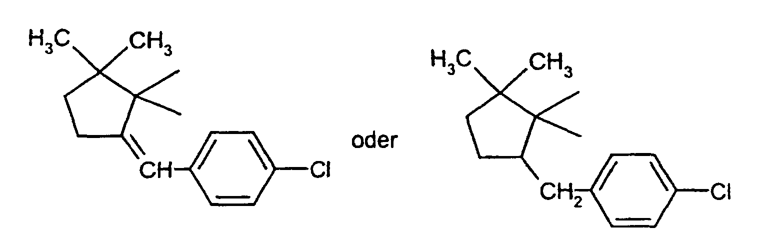
stehen,
**dadurch gekennzeichnet, dass** man Hydrazin-Derivate der Formel 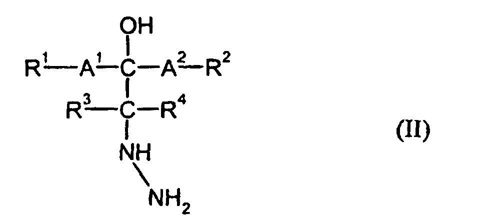 in welcher
A¹, A², R¹, R², R³ und R⁴ die oben angebenen Bedeutungen haben,
mit N-Dihalogenmethyl-formamidiniumhalogenid der Formel 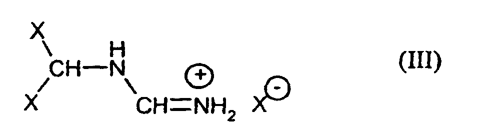 in welcher
X für Chlor oder Brom steht,
gegebenenfalls in Gegenwart eines Verdünnungsmittels umsetzt.

2. Verfahren gemäß Anspruch 1, **dadurch gekennzeichnet, dass** man Hydrazin-Derivate der Formel (II) einsetzt, in denen
A¹ für eine direkte Bindung, für gegebenenfalls durch Halogen substituiertes, geradkettiges oder verzweigtes Alkandiyl mit 1 bis 4 Kohlenstoffatomen, für gegebenenfalls durch Halogen substituiertes, geradkettiges oder verzweigtes Alkendiyl mit 2 bis 4 Kohlenstoffatomen, für gegebenenfalls durch Halogen substituiertes, geradkettiges oder verzweigtes Alkindiyl mit 2 bis 4 Kohlenstoffatomen oder für geradkettiges oder verzweigtes Alkandiyl, in dem eine Methylengruppe durch Sauerstoff ersetzt ist, mit 2 bis 4 Kettengliedern steht,
A² für eine direkte Bindung, für gegebenenfalls durch Halogen substituiertes, geradkettiges oder verzweigtes Alkandiyl mit 1 bis 6 Kohlenstoffatomen, für gegebenenfalls durch Halogen substituiertes, geradkettiges oder verzweigtes Alkendiyl mit 2 bis 6 Kohlenstoffatomen oder für gegebenenfalls durch Halogen substituiertes, geradkettiges oder verzweigtes Alkindiyl mit 2 bis 6 Kohlenstoffatomen steht,
R¹ für gegebenenfalls einfach bis dreifach, gleichartig oder verschieden durch Fluor, Chlor, Brom und/oder Alkyl mit 1 bis 4 Kohlenstoffatomen substituiertes Cycloalkyl mit 3 bis 7 Kohlenstoffatomen steht **oder**
für Aryl mit 6 bis 10 Kohlenstoffatomen steht, das einfach bis dreifach, gleichartig oder verschieden substituiert sein kann durch Fluor, Chlor, Brom, Alkyl mit 1 bis 4 Kohlenstoffatomen, Cyano, Nitro, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 9 Fluor-, Chlor- und/oder Bromatomen, Halogenalkoxy mit 1 bis 4 Kohlenstoffatomen und 1 bis 9 Fluor-, Chlor- und/oder Bromatomen oder Halogenalkylthio mit 1 bis 4 Kohlenstoffatomen und 1 bis 9 Fluor-, Chlor- und/oder Bromatomen,
R² für Wasserstoff oder für gegebenenfalls einfach bis dreifach, gleichartig oder verschieden durch Fluor, Chlor, Brom und/oder Alkyl mit 1 bis 4 Kohlenstoffatomen substituiertes Cycloalkyl mit 3 bis 7 Kohlenstoffatomen steht oder
für Aryl mit 6 bis 10 Kohlenstoffatomen steht, das einfach bis dreifach, gleichartig oder verschieden substituiert sein kann durch Fluor, Chlor, Brom, Alkyl mit 1 bis 4 Kohlenstoffatomen, Cyano, Nitro, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 9 Fluor-, Chlor- und/oder Bromatomen, Halogenalkoxy mit 1 bis 4 Kohlenstoffatomen und 1 bis 9 Fluor-, Chlor- und/oder Bromatomen oder Halogenalkylthio mit 1 bis 4 Kohlenstoffatomen und 1 bis 9 Fluor-, Chlor- und/oder Bromatomen, und
R³ und R⁴ unabhängig voneinander für Wasserstoff oder gegebenenfalls einfach bis dreifach, gleichartig oder verschieden durch Fluor, Chlor, Brom oder Alkoxy mit 1 oder 2 Kohlenstoffatomen substituiertes Alkyl mit 1 bis 4 Kohlenstoffatomen stehen oder
R³ und R⁴ gemeinsam mit dem Kohlenstoffatom, an das sie gebunden sind, für gegebenenfalls einfach bis dreifach, gleichartig oder verschieden durch Fluor, Chlor, Brom und/oder Alkyl mit 1 bis 4 Kohlenstoffatomen substituiertes Cycloalkyl mit 3 bis 6 Kohlenstoffatomen stehen,
oder
R¹, A¹ und R³ gemeinsam mit den Kohlenstoffatomen, an die sie gebunden sind, für Cycloalkyl mit 3 bis 6 Kohlenstoffatomen stehen,
A² für eine direkte Bindung steht,
R² für Wasserstoff oder für gegebenenfalls einfach bis dreifach, gleichartig oder verschieden durch Fluor, Chlor, Brom und/oder Alkyl mit 1 bis 4 Kohlenstoffatomen substituiertes Cycloalkyl mit 3 bis 7 Kohlenstoffatomen steht
oder
für Aryl mit 6 bis 10 Kohlenstoffatomen steht, das einfach bis dreifach, gleichartig oder verschieden substituiert sein kann durch Fluor, Chlor, Brom, Alkyl mit 1 bis 4 Kohlenstoffatomen, Cyano, Nitro, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 9 Fluor-, Chlor- und/oder Bromatomen, Halogenalkoxy mit 1 bis 4 Kohlenstoffatomen und 1 bis 9 Fluor-, Chlor- und/oder Bromatomen oder Halogenalkylthio mit 1 bis 4 Kohlenstoffatomen und 1 bis 9 Fluor-, Chlor- und/oder Bromatomen, und
R⁴ für Wasserstoff oder gegebenenfalls einfach bis dreifach, gleichartig oder verschieden durch Fluor, Chlor, Brom oder Alkoxy mit 1 oder 2 Kohlenstoffatomen substituiertes Alkyl mit 1 bis 4 Kohlenstoffatomen steht,
oder
R³ und R⁴ für Wasserstoff stehen und die Gruppen
R¹-A¹- und R²-A²- gemeinsam mit dem Kohlenstoffatom, an das sie gebunden sind, für einen Rest der Formel 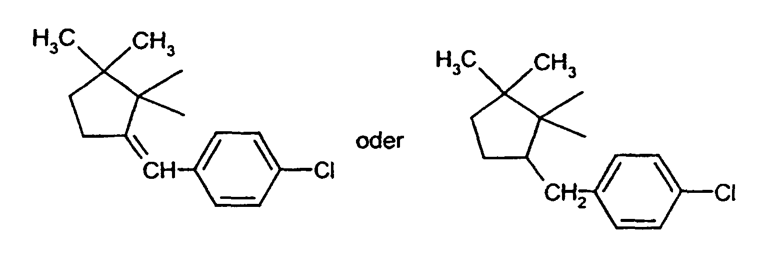
stehen.

3. Verfahren gemäß Anspruch 1, **dadurch gekennzeichnet, dass** man Hydrazin-Derivate der Formel (II) einsetzt, in denen
A¹ für eine Einfachbindung, für Methylen, Ethan-1,1-diyl, Ethan-1,2-diyl, Ethen-1,2-diyl, Ethin-1,2-diyl oder -O-CH₂ steht, wobei die CH₂-Gruppe mit dem Carbinol-Kohlenstoffatom verbunden ist,
A² für eine direkte Bindung steht oder für jeweils gegebenenfalls einfach oder zweifach durch Fluor und/oder Chlor substituiertes Methylen, Ethan-1,1-diyl, Ethan-1,2-diyl, Propan-1,1-diyl, Propan-1,2-diyl, Propan-1,3-diyl, Propan-2,2-diyl, Butan-1,1-diyl, Butan-1,2-diyl, Butan-1,3-diyl, Butan-1,4-diyl, Butan-2,2-diyl, 2-Methyl-propan-1,2-diyl, Ethylen-1,2-diyl oder Ethin-1,2-diyl steht,
R¹ für gegebenenfalls einfach bis dreifach, gleichartig oder verschieden durch Fluor, Chlor, Brom, Methyl, Ethyl, n-Propyl, i-Propyl, n-Butyl, iso-Butyl, sek.-Butyl oder tert.-Butyl substituiertes Cycloalkyl mit 3 bis 6 Kohlenstoffatomen steht oder
für Phenyl oder Naphthyl steht, das jeweils einfach bis dreifach, gleichartig oder verschieden substituiert sein kann durch Fluor, Chlor, Brom, Cyano, Nitro, Methyl, Ethyl, n-Propyl, i-Propyl, n-Butyl, sek.-Butyl, iso-Butyl, tert.-Butyl, Methoxy, Ethoxy, Methylthio, Ethylthio, Trifluormethyl, Trifluormethoxy, Difluormethoxy, Trifluormethylthio und/oder Difluonnethylthio,
R² für Wasserstoff oder für gegebenenfalls einfach bis dreifach, gleichartig oder verschieden durch Fluor, Chlor, Brom, Methyl, Ethyl, n-Propyl, i-Propyl, n-Butyl, iso-Butyl, sek.-Butyl oder tert.-Butyl substituiertes Cycloalkyl mit 3 bis 6 Kohlenstoffatomen steht oder für Phenyl oder Naphthyl steht, das jeweils einfach bis dreifach, gleichartig oder verschieden substituiert sein kann durch Fluor, Chlor, Brom, Cyano, Nitro, Methyl, Ethyl, n-Propyl, i-Propyl, n-Butyl, sek.-Butyl, iso-Butyl, tert.-Butyl, Methoxy, Ethoxy, Methylthio, Ethylthio, Trifluormethyl, Trifluormethoxy, Difluormethoxy, Trifluormethylthio und/oder Difluonnethylthio,
R³ und R⁴ unabhängig voneinander für Wasserstoff, Methyl oder Ethyl stehen oder
R³ und R⁴ gemeinsam mit dem Kohlenstoffatom, an das sie gebunden sind, für Cyclopropyl, Cyclobutyl, Cyclopentyl oder Cyclohexyl stehen
oder
R¹, A¹ und R³ gemeinsam mit den Kohlenstoffatomen, an die sie gebunden sind, für Cyclopropyl, Cyclobutyl, Cyclopentyl oder Cyclohexyl stehen,
A² für eine direkte Bindung steht,
R² für Wasserstoff oder
für gegebenenfalls einfach bis dreifach, gleichartig oder verschieden durch Fluor, Chlor, Brom, Methyl, Ethyl, n-Propyl, i-Propyl, n-Butyl, iso-Butyl, sek.-Butyl oder tert.-Butyl substituiertes Cycloalkyl mit 3 bis 6 Kohlenstoffatomen steht, oder
für Phenyl oder Naphthyl steht, das jeweils einfach bis dreifach, gleichartig oder verschieden substituiert sein kann durch Fluor, Chlor, Brom, Cyano, Nitro, Methyl, Ethyl, n-Propyl, i-Propyl, n-Butyl, sek.-Butyl, iso-Butyl, tert.-Butyl, Methoxy, Ethoxy, Methylthio, Ethylthio, Trifluormethyl, Trifluormethoxy, Difluormethoxy, Trifluormethylthio und/oder Difluonnethylthio und
R⁴ für Wasserstoff, Methyl oder Ethyl steht,
oder
R³ und R⁴ für Wasserstoff stehen und die Gruppen
R¹-A¹- und R²-A²- gemeinsam mit dem Kohlenstoffatom, an das sie gebunden sind, für einen Rest der Formel 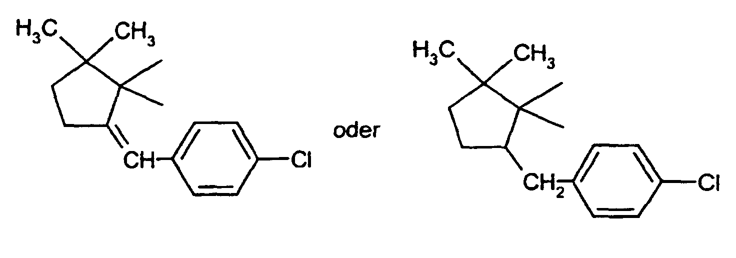
stehen.

4. Verfahren gemäß Anspruch 1, **dadurch gekennzeichnet, dass** man Hydrazin-Derivate der Formel (II) in Form von Säureadditions-Salzen einsetzt.

5. Verfahren gemäß Anspruch 1, **dadurch gekennzeichnet, dass** man N-Dichlormethyl-formamidinium-hydrochlorid als Reaktionskomponente der Formel (III) einsetzt.

6. Verfahren gemäß Anspruch 1, **dadurch gekennzeichnet, dass** man bei Temperaturen zwischen 20°C und 200°C arbeitet.

## Claims

1. Process for preparing 2-(1,2,4-triazol-1-yl)-ethanols of the formula 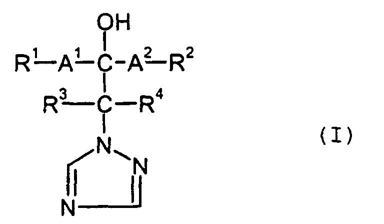 in which
A¹ and A² independently of one another represent a direct bond, represent optionally halogen-substituted alkanediyl, represent optionally halogen-substituted alkenediyl, represented optionally halogen-substituted alkinediyl or represent alkanediyl in which a methylene group is replaced by oxygen,
R¹ and R² independently of one another represent hydrogen, represent optionally substituted cycloalkyl or represent optionally substituted aryl and
R³ and R⁴ independently of one another represent hydrogen or represent optionally substituted alkyl or
R³ and R⁴ together with the carbon atom to which they are attached represent optionally substituted cycloalkyl,
or
R¹, A¹ and R³ together with the carbon atoms to which they are attached represent cycloalkyl,
A² represents a direct bond, represents optionally halogen-substituted alkanediyl, represents optionally halogen-substituted alkenediyl, represents optionally halogen-substituted alkinediyl or represents alkanediyl in which one methylene group has been replaced by oxygen,
R² represents hydrogen, represents optionally substituted cycloalkyl or represents optionally substituted aryl and
R⁴ represents hydrogen or represents optionally substituted alkyl,
or
R³ and R⁴ represent hydrogen and the groups
R¹-A¹- and R²-A²- together with the carbon atom to which they are attached represent a radical of the formula 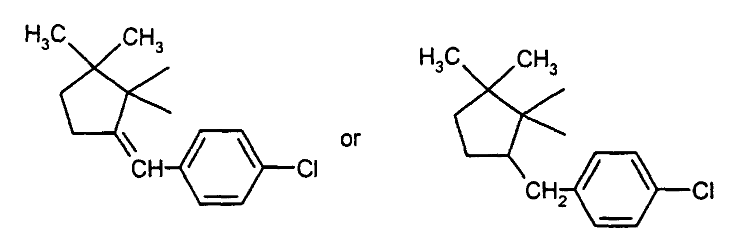
**characterized in that** hydrazine derivatives of the formula 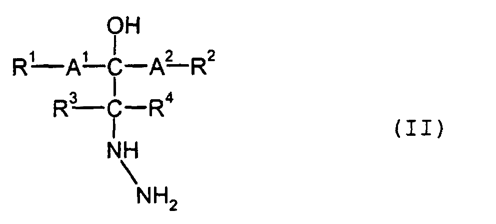 in which
A¹, A², R¹, R², R³ and R⁴ are as defined above
are reacted with N-dihalogenomethyl-formamidinium halide of the formula 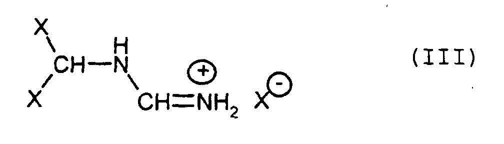 in which
X represents chlorine or bromine,
if appropriate in the presence of a diluent.

2. Process according to Claim 1, **characterized in that** hydrazine derivatives of the formula (II) are used in which
A¹ represents a direct bond, represents optionally halogen-substituted, straight-chain or branched alkanediyl having 1 to 4 carbon atoms, represents optionally halogen-substituted, straight-chain or branched alkenediyl having 2 to 4 carbon atoms, represents optionally halogen-substituted straight-chain or branched alkinediyl having 2 to 4 carbon atoms or represents straight-chain or branched alkanediyl in which one methylene group has been replaced by oxygen, having 2 to 4 chain members,
A² represents a direct bond, represents optionally halogen-substituted straight-chain or branched alkanediyl having 1 to 6 carbon atoms, represents optionally halogen-substituted straight-chain or branched alkenediyl having 2 to 6 carbon atoms or represents optionally halogen-substituted straight-chain or branched alkinediyl having 2 to 6 carbon atoms,
R¹ represents cycloalkyl having 3 to 7 carbon atoms which is optionally mono- to trisubstituted by identical or different substituents from the group consisting of fluorine, chlorine, bromine and/or alkyl having 1 to 4 carbon atoms or
represents aryl having 6 to 10 carbon atoms which may be mono- to trisubstituted by identical or different substituents from the group consisting of fluorine, chlorine, bromine, alkyl having 1 to 4 carbon atoms, cyano, nitro, alkoxy having 1 to 4 carbon atoms, alkylthio having 1 to 4 carbon atoms, halogenoalkyl having 1 to 4 carbon atoms and 1 to 9 fluorine, chlorine and/or bromine atoms, halogenoalkoxy having 1 to 4 carbon atoms and 1 to 9 fluorine, chlorine and/or bromine atoms or halogenoalkylthio having 1 to 4 carbon atoms and 1 to 9 fluorine, chlorine and/or bromine atoms,
R² represents hydrogen or represents cycloalkyl having 3 to 7 carbon atoms which is optionally mono- to trisubstituted by identical or different substituents from the group consisting of fluorine, chlorine, bromine and alkyl having 1 to 4 carbon atoms or
represents aryl having 6 to 10 carbon atoms which may be mono- to trisubstituted by identical or different substituents from the group consisting of fluorine, chlorine, bromine, alkyl having 1 to 4 carbon atoms, cyano, nitro, alkoxy having 1 to 4 carbon atoms, alkylthio having 1 to 4 carbon atoms, halogenoalkyl having 1 to 4 carbon atoms and 1 to 9 fluorine, chlorine and/or bromine atoms, halogenoalkoxy having 1 to 4 carbon atoms and 1 to 9 fluorine, chlorine and/or bromine atoms and halogenoalkylthio having 1 to 4 carbon atoms and 1 to 9 fluorine, chlorine and/or bromine atoms, and
R³ and R⁴ independently of one another represent hydrogen or alkyl having 1 to 4 carbon atoms which is optionally mono- to trisubstituted by identical or different substituents from the group consisting of fluorine, chlorine, bromine and alkoxy having 1 or 2 carbon atoms or
R³ and R⁴ together with the carbon atom to which they are attached represent cycloalkyl having 3 to 6 carbon atoms which is optionally mono- to trisubstituted by identical or different substituents from the group consisting of fluorine, chlorine, bromine and alkyl having 1 to 4 carbon atoms,
or
R¹, A¹ and R³ together with the carbon atoms to which they are attached represent cycloalkyl having 3 to 6 carbon atoms,
A² represents a direct bond,
R² represents hydrogen or represents cycloalkyl having 3 to 7 carbon atoms which is optionally mono- to trisubstituted by identical or different substituents from the group consisting of fluorine, chlorine, bromine and alkyl having 1 to 4 carbon atoms or
represents aryl having 6 to -10 carbon atoms which may be mono- to trisubstituted by identical or different substituents from the group consisting of fluorine, chlorine, bromine, alkyl having 1 to 4 carbon atoms, cyano, nitro, alkoxy having 1 to 4 carbon atoms, alkylthio having 1 to 4 carbon atoms, halogenoalkyl having 1 to 4 carbon atoms and 1 to 9 fluorine, chlorine and/or bromine atoms, halogenoalkoxy having 1 to 4 carbon atoms and 1 to 9 fluorine, chlorine and/or bromine atoms or halogenoalkylthio having 1 to 4 carbon atoms and 1 to 9 fluorine, chlorine and/or bromine atoms, and
R⁴ represents hydrogen or represents alkyl having 1 to 4 carbon atoms which is optionally mono- to trisubstituted by identical or different substituents from the group consisting of fluorine, chlorine, bromine and alkoxy having 1 or 2 carbon atoms,
or
R³ and R⁴ represent hydrogen and the groups
R¹-A¹- and R²-A²- together with the carbon atoms to which they are attached represent a radical of the formula

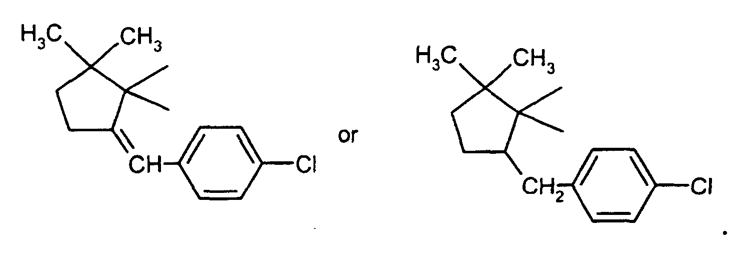

3. Process according to Claim 1, **characterized in that** hydrazine derivatives of the formula (II) are used in which
A¹ represents a single bond, represents methylene, ethane-1,1-diyl, ethane-1,2-diyl, ethene-1,2-diyl, ethine-1,2-diyl or -O-CH₂, where the CH₂ group is attached to the carbinol carbon atom,
A² represents a direct bond or represents methylene, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-1,4-diyl, butane-2,2-diyl, 2-methyl-propane-1,2-diyl, ethylene-1,2-diyl or ethine-1,2-diyl, each of which is optionally mono- or disubstituted by fluorine and/or chlorine,
R¹ represents cycloalkyl having 3 to 6 carbon atoms which is optionally mono- to trisubstituted by identical or different substituents from the group consisting of fluorine, chlorine, bromine, methyl, ethyl, n-propyl, i-propyl, n-butyl, iso-butyl, sec-butyl and tert-butyl or
represents phenyl or naphthyl, each of which may be mono- to trisubstituted by identical or different substituents from the group consisting of fluorine, chlorine, bromine, cyano, nitro, methyl, ethyl, n-propyl, i-propyl, n-butyl, sec-butyl, iso-butyl, tert-butyl, methoxy, ethoxy, methylthio, ethylthio, trifluoromethyl, trifluoromethoxy, difluoromethoxy, trifluoromethylthio and difluoromethylthio,
R² represents hydrogen or represents cycloalkyl having 3 to 6 carbon atoms which is optionally mono- to trisubstituted by identical or different substituents from the group consisting of fluorine, chlorine, bromine, methyl, ethyl, n-propyl, i-propyl, n-butyl, iso-butyl, sec-butyl and tert-butyl or
represents phenyl or naphthyl, each of which may be mono- to trisubstituted by identical or different substituents from the group consisting of fluorine, chlorine, bromine, cyano, nitro, methyl, ethyl, n-propyl, i-propyl, n-butyl, sec-butyl, iso-butyl, tert-butyl, methoxy, ethoxy, methylthio, ethylthio, trifluoromethyl, trifluoromethoxy, difluoromethoxy, trifluoromethylthio and difluoromethylthio,
R³ and R⁴ independently of one another represent hydrogen, methyl or ethyl or
R³ and R⁴ together with the carbon atom to which they are attached represent cyclopropyl, cyclobutyl, cyclopentyl or cyclohexyl
or
R¹, A¹ and R³ together with the carbon atoms to which they are attached represent cyclopropyl, cyclobutyl, cyclopentyl or cyclohexyl,
A² represents a direct bond
R² represents hydrogen or
represents cycloalkyl having 3 to 6 carbon atoms which is optionally mono- to trisubstituted by identical or different substituents from the group consisting of fluorine, chlorine, bromine, methyl, ethyl, n-propyl, i-propyl, n-butyl, iso-butyl, sec-butyl and tert-butyl, or
represents phenyl or naphthyl, each of which may be mono- to trisubstituted by identical
or different substituents from the group consisting of fluorine, chlorine, bromine, cyano, nitro, methyl, ethyl, n-propyl, i-propyl, n-butyl, sec-butyl, iso-butyl, tert-butyl, methoxy, ethoxy, methylthio, ethylthio, trifluoromethyl, trifluoromethoxy, difluoromethoxy, trifluoromethylthio and difluoromethylthio and
R⁴ represents hydrogen, methyl or ethyl,
or
R³ and R⁴ represent hydrogen and the groups
R¹-A¹- and R²-A²- together with the carbon atom to which they are attached represent a radical of the formula

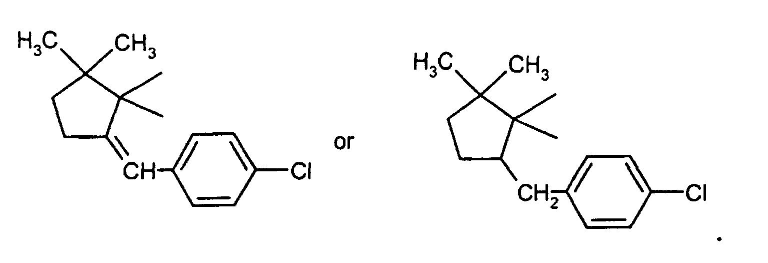

4. Process according to Claim 1, **characterized in that** hydrazine derivatives of the formula (II) are employed in the form of acid addition salts.

5. Process according to Claim 1, **characterized in that** the reaction component of the formula (III) used is N-dichloromethyl-formamidinium hydrochloride.

6. Process according to Claim 1, **characterized in that** it is carried out at temperatures between 20°C and 200°C.

## Revendications

1. Procédé de préparation de 2-(1,2,4-triazol-1-yl)éthanols de la formule : 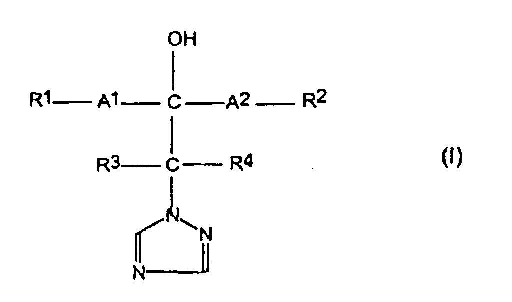 dans laquelle :
A¹ et A² représentent indépendamment l'un de l'autre, une liaison directe, un radical alcanediyle le cas échéant substitué par un atome d'halogène, un radical alcènediyle le cas échéant substitué par un atome d'halogène, un radical alcynediyle le cas échéant substitué par un atome d'halogène, ou un radical alcanediyle dans lequel un radical méthylène est remplacé par un atome d'oxygène,
R¹ et R² représentent indépendamment l'un de l'autre, l'atome d'hydrogène, un radical cycloalcoyle le cas échéant substitué ou un radical aryle le cas échéant substitué, et
R³ et R⁴ représentent indépendamment l'un de l'autre, l'atome d'hydrogène, un radical alcoyle le cas échéant substitué, ou
R³ et R⁴ représentent avec l'atome de carbone, sur lequel ils sont liés, un radical cycloalcoyle le cas échéant substitué,
ou
R¹, A¹ et R³ représentent avec l'atome de carbone, sur lequel ils sont liés, un radical cycloalcoyle,
A² représente une liaison directe, un radical alcanediyle le cas échéant substitué par un atome d'halogène, un radical alcènediyle le cas échéant substitué par un atome d'halogène, un radical alcynediyle le cas échéant substitué par un atome d'halogène, ou un radical alcanediyle dans lequel un radical méthylène est remplacé par un atome d'oxygène,
R² représente l'atome d'hydrogène, un radical cycloalcoyle le cas échéant substitué ou un radical aryle le cas échéant substitué, et
R⁴ représente l'atome d'hydrogène, un radical alcoyle le cas échéant substitué,
ou
R³ et R⁴ représentent l'atome d'hydrogène, et les groupements
R¹-A¹- et R²-A²- représentent avec l'atome de carbone, sur lequel ils sont liés, un reste de la formule : 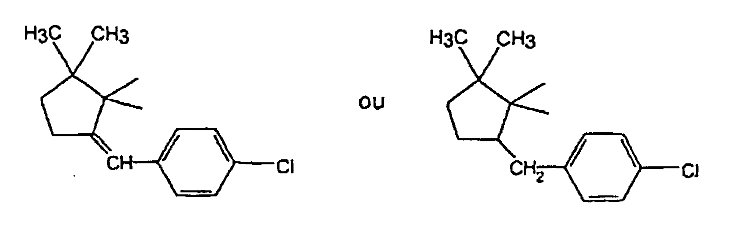
**caractérisé en ce que** l'on fait réagir un dérivé de l'hydrazine de la formule : 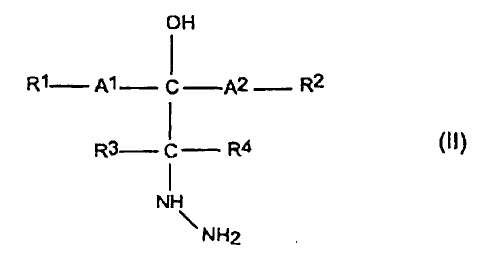 dans laquelle :
A¹, A², R¹, R², R³ et R⁴ ont les significations indiquées ci-dessus,
avec un halogénure de N-dihalogénométhylformamidinium de la formule : 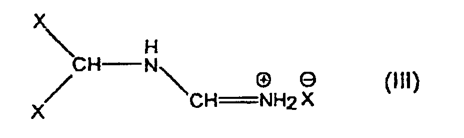
dans laquelle
X représente l'atome de chlore ou de brome,
le cas échéant en présence d'un agent de dilution.

2. Procédé suivant la revendication 1, **caractérisé en ce que** l'on met en oeuvre des dérivés de l'hydrazine de la formule (II), dans laquelle :
A¹ représente une liaison directe, un radical alcanediyle linéaire ou ramifié, le cas échéant substitué par un atome d'halogène, ayant 1 à 4 atomes de carbone, un radical alcènediyle linéaire ou ramifié, le cas échéant substitué par un atome d'halogène, ayant 2 à 4 atomes de carbone, un radical alcynediyle linéaire ou ramifié, le cas échéant substitué par un atome d'halogène, ayant 2 à 4 atomes de carbone, ou un radical alcanediyle dans lequel un radical méthylène est remplacé par un atome d'oxygène, avec 2 à 4 membres de chaine,
A² représente une liaison directe, un radical alcanediyle linéaire ou ramifié, le cas échéant substitué par un atome d'halogène, ayant 1 à 6 atomes de carbone, un radical alcènediyle linéaire ou ramifié, le cas échéant substitué par un atome d'halogène, ayant 2 à 6 atomes de carbone, ou un radical alcynediyle linéaire ou ramifié, le cas échéant substitué par un atome d'halogène, ayant 2 à 6 atomes de carbone,
R¹ représente un radical cycloalcoyle ayant 3 à 7 atomes de carbone, le cas échéant substitué une à trois fois, de manière identique ou différente, par l'atome de fluor, de chlore, de brome et/ou un radical alcoyle ayant 1 à 4 atomes de carbone, ou représente un radical aryle ayant 6 à 10 atomes de carbone, qui peut être substitué une à trois fois, de manière identique ou différente, par l'atome de fluor, de chlore, de brome, un radical alcoyle ayant 1 à 4 atomes de carbone, le radical cyano, nitro, un radical alcoxy ayant 1 à 4 atomes de carbone, un radical alcoylthio ayant 1 à 4 atomes de carbone, un radical halogénoalcoyle ayant 1 à 4 atomes de carbone et 1 à 9 atomes de fluor, de chlore et/ou de brome, un radical halogénoalcoxy ayant 1 à 4 atomes de carbone et 1 à 9 atomes de fluor, de chlore et/ou de brome, ou un radical halogénoalcoylthio ayant 1 à 4 atomes de carbone et 1 à 9 atomes de fluor, de chlore et/ou de brome,
R² représente l'atome d'hydrogène ou un radical cycloalcoyle ayant 3 à 7 atomes de carbone, le cas échéant substitué une à trois fois, de manière identique ou différente, par l'atome de fluor, de chlore, de brome et/ou un radical alcoyle ayant 1 à 4 atomes de carbone, ou représente un radical aryle ayant 6 à 10 atomes de carbone, qui peut être substitué une à trois fois, de manière identique ou différente, par l'atome de fluor, de chlore, de brome, un radical alcoyle ayant 1 à 4 atomes de carbone, le radical cyano, nitro, un radical alcoxy ayant 1 à 4 atomes de carbone, un radical alcoylthio ayant 1 à 4 atomes de carbone, un radical halogénoalcoyle ayant 1 à 4 atomes de carbone et 1 à 9 atomes de fluor, de chlore et/ou de brome, un radical halogénoalcoxy ayant 1 à 4 atomes de carbone et 1 à 9 atomes de fluor, de chlore et/ou de brome, ou un radical halogénoalcoylthio ayant 1 à 4 atomes de carbone et 1 à 9 atomes de fluor, de chlore et/ou de brome, et
R³ et R⁴ représentent indépendamment l'un de l'autre, l'atome d'hydrogène ou un radical alcoyle ayant de 1 à 4 atomes de carbone, le cas échéant substitué une à trois fois, de manière identique ou différente, par l'atome de fluor, de chlore, de brome et/ou un radical alcoxy ayant 1 ou 2 atomes de carbone, ou
R³ et R⁴ représentent avec l'atome de carbone, sur lequel ils sont liés, un radical cycloalcoyle ayant 3 à 6 atomes de carbone, le cas échéant substitué une à trois fois, de manière identique ou différente, par l'atome de fluor, de chlore, de brome et/ou un radical alcoyle ayant 1 à 4 atomes de carbone,
ou
R¹, A¹ et R³ représentent avec l'atome de carbone, sur lequel ils sont liés, un radical cycloalcoyle ayant 3 à 6 atomes de carbone,
A² représente une liaison directe,
R² représente l'atome d'hydrogène ou un radical cycloalcoyle ayant 3 à 7 atomes de carbone, le cas échéant substitué une à trois fois, de manière identique ou différente, par l'atome de fluor, de chlore, de brome et/ou un radical alcoyle ayant 1 à 4 atomes de carbone, ou représente un radical aryle ayant 6 à 10 atomes de carbone, qui peut être substitué une à trois fois, de manière identique ou différente, par l'atome de fluor, de chlore, de brome, un radical alcoyle ayant 1 à 4 atomes de carbone, le radical cyano, nitro, un radical alcoxy ayant 1 à 4 atomes de carbone, un radical alcoylthio ayant 1 à 4 atomes de carbone, un radical halogénoalcoyle ayant 1 à 4 atomes de carbone et 1 à 9 atomes de fluor, de chlore et/ou de brome, un radical halogénoalcoxy ayant 1 à 4 atomes de carbone et 1 à 9 atomes de fluor, de chlore et/ou de brome, ou un radical halogénoalcoylthio ayant 1 à 4 atomes de carbone et 1 à 9 atomes de fluor, de chlore et/ou de brome, et
R⁴ représente l'atome d'hydrogène ou un radical alcoyle ayant de 1 à 4 atomes de carbone, le cas échéant substitué une à trois fois, de manière identique ou différente, par l'atome de fluor, de chlore, de brome et/ou un radical alcoxy ayant 1 ou 2 atomes de carbone,
ou
R³ et R⁴ représentent l'atome d'hydrogène, et les groupements
R¹-A¹- et R²-A²- représentent avec l'atome de carbone, sur lequel ils sont liés, un reste de la formule :

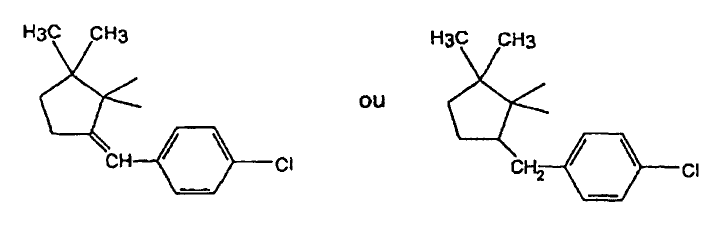

3. Procédé suivant la revendication 1, **caractérisé en ce que** l'on met en oeuvre des dérivés de l'hydrazine de la formule (II), dans laquelle :
A¹ représente une liaison simple, le radical méthylène, éthane-1,1-diyle, éthane-1,2-diyle, éthène-1,2-diyle, éthyne-1,2-diyle ou -O-CH₂, où le radical CH₂ est relié à l'atome de carbone du carbinol,
A² représente une liaison directe, le radical méthylène, éthane-1,1-diyle, éthane-1,2-diyle, propane-1,1-diyle, propane-1,2-diyle, propane-1,3-diyle, propane-2,2-diyle, butane-1,1-diyle, butane-1,2-diyle, butane-1,3-diyle, butane-1,4-diyle, butane-2,2-diyle, 2-méthylpropane-1,2-diyle, éthène-1,2-diyle ou éthyne-1,2-diyle, chaque fois le cas échéant substitué une ou deux fois, par l'atome de fluor et/ou de chlore,
R¹ représente un radical cycloalcoyle ayant 3 à 6 atomes de carbone, le cas échéant substitué une à trois fois, de manière identique ou différente, par l'atome de fluor, de chlore, de brome, le radical méthyle, éthyle, n-propyle, i-propyle, n-butyle, i-butyle, s-butyle ou t-butyle, ou représente le radical phényle ou naphtyle, qui peut être chaque fois substitué une à trois fois, de manière identique ou différente, par l'atome de fluor, de chlore, de brome, le radical cyano, nitro, méthyle, éthyle, n-propyle, i-propyle, n-butyle, i-butyle, s-butyle, t-butyle, méthoxy, éthoxy, méthylthio, éthylthio, trifluorométhyle, trifluorométhoxy, difluorométhoxy, trifluorométhylthio et/ou difluorométhylthio,
R² représente l'atome d'hydrogène ou un radical cycloalcoyle ayant 3 à 6 atomes de carbone, le cas échéant substitué une à trois fois, de manière identique ou différente, par l'atome de fluor, de chlore, de brome, le radical méthyle, éthyle, n-propyle, i-propyle, n-butyle, i-butyle, s-butyle ou t-butyle, ou représente le radical phényle ou naphtyle, qui peut être chaque fois substitué une à trois fois, de manière identique ou différente, par l'atome de fluor, de chlore, de brome, le radical cyano, nitro, méthyle, éthyle, n-propyle, i-propyle. n-butyle, i-butyle, s-butyle, t-butyle, méthoxy, éthoxy, méthylthio, éthylthio, trifluorométhyle, trifluorométhoxy, difluorométhoxy, trifluorométhylthio et/ou difluorométhylthio,
R³ et R⁴ représentent indépendamment l'un de l'autre, l'atome d'hydrogène, le radical méthyle ou éthyle, ou
R³ et R⁴ représentent avec l'atome de carbone, sur lequel ils sont liés, le radical cyclopropyle, cyclobutyle, cyclopentyle ou cyclohexyle,
ou
R¹, A¹ et R³ représentent avec l'atome de carbone, sur lequel ils sont liés, le radical cyclopropyle, cyclobutyle, cyclopentyle ou cyclohexyle,
A² représente une liaison directe,
R² représente l'atome d'hydrogène ou un radical cycloalcoyle ayant 3 à 6 atomes de carbone, le cas échéant substitué une à trois fois, de manière identique ou différente, par l'atome de fluor, de chlore, de brome, le radical méthyle, éthyle, n-propyle, i-propyle, n-butyle, i-butyle, s-butyle ou t-butyle, ou représente le radical phényle ou naphtyle, qui peut être chaque fois substitué une à trois fois, de manière identique ou différente, par l'atome de fluor, de chlore, de brome, le radical cyano, nitro, méthyle, éthyle, n-propyle, i-propyle, n-butyle, i-butyle, s-butyle, t-butyle, méthoxy, éthoxy, méthylthio, éthylthio, trifluorométhyle, trifluorométhoxy, difluorométhoxy, trifluorométhylthio et/ou difluorométhylthio, et
R⁴ représente l'atome d'hydrogène, le radical méthyle ou éthyle,
ou
R³ et R⁴ représentent l'atome d'hydrogène, et les groupements
R¹-A¹- et R²-A²- représentent avec l'atome de carbone, sur lequel ils sont liés, un reste de la formule :

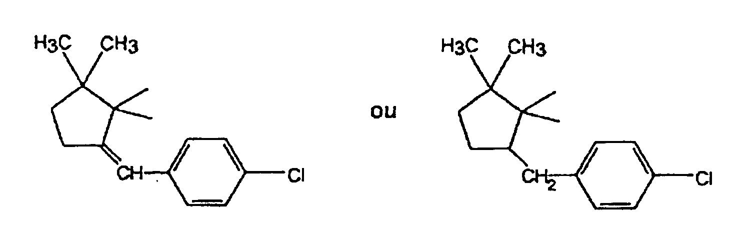

4. Procédé suivant la revendication 1, **caractérisé en ce que** l'on met en oeuvre des dérivés de l'hydrazine de la formule (II) sous la forme de sels d'addition d'acide.

5. Procédé suivant la revendication 1, **caractérisé en ce que** l'on met en oeuvre le chlorure de N-dichlorométhylformamidinium comme composant de réaction de la formule (III).

6. Procédé suivant la revendication 1, **caractérisé en ce que** l'on travaille à des températures allant de 2D°C à 200°C.
